# EUROPEAN PATENT APPLICATION

(11) **EP 3 438 624 A1**
(43) Date of publication of application: **06.02.2019**
(21) Application number: 17184576.1
(22) Date of filing: 02.08.2017
(51) Int. Cl.: G01J 3/44, G01J 3/453, G01N 33/02, G01N 21/64, G01J 3/02

(54) **DEVICE AND METHOD FOR REDUCED SPECTRAL FLUORESCENCE SPECTROSCOPY**

(71) Applicant: SENMARK INVEST OÜ, 11415 Tallinn (EE)
(72) Inventor: TAMM, Martti, 10617 Tallinn (EE); SÕRMUS, Aavo, 76401 Laagri, Harju maakond (EE); TIISMA, Kalle, 11620 Tallinn (EE); LOOV, Roman, 74001 Haabneeme, Harju maakond (EE)
(74) Representative: Sarap, Margus

(57) **Abstract**

A device to perform Reduced Spectral Fluorescence Spectroscopy (RSFS) with no moving parts, where multi-wavelength diode array (2) with bandpass filters (3) performs relevant excitation of a medium and photomultiplier (7) with applied filter system (6) and detects the emission spectra. The excitation radiation to the medium and emission radiation from medium is delivered with an optical fiber probe (8) with a special configuration to guarantee even distribution both excitation and emission spectra. Excitation and emission spectra are modulated so that the backlight will not disturb the measurement, furthermore the modulated signal system sensitivity is good enough for the measurement to take place in a distance up to 10 cm. As excitation diode array has limited number of excitation wavelengths, changeable diode arrays with corresponding emission filter sets can be exchanged with respect to medium which is being measured. Corresponding databases for different excitation diode arrays with corresponding filter sets enable to calculate parameters of the medium what single wavelength fluorimeters can't do.

## Description

### Field of the invention

The present invention belongs to the field of fluorescence spectroscopy.

### Prior art

Fluorescence Spectroscopy is a powerful tool to describe various foodstuffs. Fluorescence refers to cold light emission by electron transfer due to molecule energy state change. Molecules are excited by photons with a different energy state. Fluorescence spectroscopy is unique among other spectroscopic techniques because of its multidimensional nature, fluorophore can be exited only with specific energy which results to longer wavelength emission photon.

There are wide range of naturally fluorescent compounds occurring in foodstuffs, among others aromatic amino acids, flavonoids, vitamins. Everything mentioned have their dedicated excitation and emission parameters if standard solution of the material is made, unfortunately this is not the case of real life situation. Several factors can affect emission spectra, concentration, local molecular environment. Occurring quenching effect is either static or dynamic. Static quenching occurs when formation of the excited state is inhibited due to a ground-state complex formation in which the fluorophore forms non-fluorescent complexes with a quencher molecule. Dynamic or collisional quenching refers to the process when a quencher interferes with (deactivates) the behavior of the excited state after its formation. The excited molecule will be deactivated by contact with other molecules or by intra- or intermolecular interactions. Higher temperatures increase collisional quenching due to the increased velocities of the molecules. Resonance energy transfer can also be considered as a kind of dynamic quenching. Resonance energy transfer occurs when the emission spectrum of a fluorophore overlaps with the absorption spectrum of an acceptor molecule. The energy transfer does not involve emission of light, but rather a direct interaction between the donor and acceptor molecule, leading to a full or partial deactivation of the exited fluorophore. (Christensen, Norgaard, Bro, & Engelsen, 2006)

Several scientific sources state that correlation databases based on Spectral Fluorecence Signature (SFS) spectra can be built despite the occurring quenching effect (Allais, Viaud, Pierre, & Dufour, 2004; Hassoun & Karoui, 2016). The use of statistical tools is necessary to extract correlation from multidimensional spectra. Usually SFS spectra is a ∼50x50 data points matrix from which the correlation databases are built, if the parameter of the medium is measurable with SFS.

SFS spectroscopy is widely used as a laboratory method to carry out different quality studies on waste water, foodstuffs etc (Ahmad, Nache, Waffenschmidt, & Hitzmann, 2016; Elmasry et al., 2015; Lopez & Dufour, 2001). Together with modern statistical analyses tools correlation models for different mediums can be generated to measure various quality parameters; contamination, food fraud, milk coagulation to name a few. Multi-dimensional datasets, such as SFS plots, require multi-dimensional statistical tools (PCA, PARAFAC) to look for the biggest correlation coefficients between measured datasets and reference methods (milk fat, food fraud, milk coagulation etc.). Usually 4 generated maximum difference dimensions are enough to have a working correlation model if SFS can be used as a method to describe parameter of a medium. Generated maximum difference dimensions correspond to ex/em wavelength pairs.

All laboratory spectroscopy devices have one thing in common, they all have moving parts; mirror, diffraction grating, this makes the equipment fragile. In most cases calibration procedure is needed after relocation of the device. Also, laboratory devices use measurement setup where complete separation of ambient light during measurement is achieved with a closable measurement chambers and cuvettes.

Another type of problems known from prior art are following. There are robust single wavelength fluorimeters used in many applications. Usually a diode excitation is used and photomultiplier as a detector. Many known systems have corresponding optical filters engaged into the system to guarantee specific excitation/emission pairs. Advantage of fluorimeters is continuous measurement possibility, disadvantage is quenching process during measurement in a medium. Known systems can't provide quantitative results with the measurement of only one wavelength if optical density of the medium is OD>0,15. In such occasion multiple wavelengths must be measured to from the correlation model for adequate medium description as described before.

For example, US patent US4750837A describes a fluorimeter which can measure small proportions of fluorescence. This system does not consider the quenching effect.

The closest prior art to the present invention is known for example from US Patent US8872133. The patent describes a device and method for measuring the fluorescence of milk to determine the protein content. The intensity of the fluorescence depends on concentration but complex mixtures, such as food, the fluorescence may not be additive. In fact, the correlation between single excitation/emission spectra and concentration of the fluorophore is linear if the optical density (OD) is less than 0,15. If to try to measure protein content as described in patent US8872133 it is not possible in real life. The concentration range of the fluorophores must be in linear quenching free range if one wants to quantify the measurement. Signals from fluorophores must be independent from each other, and signal contributions from interferents such as absorbing and quenching must be insignificant compared to the fluorescence signal. (Christensen et al., 2006) Such conditions are not fulfilled in most foodstuffs (especially in milk what was mentioned in the patent) and concentration measurement as described in patent US8872133 is impossible.

In dairy products fat is always present together with vitamin-A. Vitamin-A excitation frequency overlaps with emission spectra of tryptophan. With the measurement of only one ex/em pair protein content measurement is impossible as fluorescence intensity is influenced by both; fat content and protein content.

### Brief description of the invention

The aim of the present invention is to provide a device and method to identify and quantify substances in real-time by the measurement of Reduced Spectral Fluorescence Spectroscopy, which would be durable and without moving parts, free of quenching problem, quickly adjustable, enabling to measure multiple wavelengths at the same time. The system is applicable to milk fat content measurement, capsaicin content in chili peppers, jam content in dairy products to name a few.

The aim according to the present invention is achieved by a device combining robustness and complex measurement to perform continuous online measurements. The device according to present invention measures spectrums whereas known fluorimeters measure only single Ex/Em wavelength pair. Modulation of signals enables RSFS measurement to take place at the presence of ambient light which is unique among known spectral fluorescence spectroscopy devices.

Compared to the known spectral fluorescence signature devices the device described in the present patent application has no moving parts and does not need a cuvette to perform a measurement. Furthermore, the modulation of the signal of the present device makes it possible to measure SFS spectrums at the presence of ambient light and enables measurements from distance.

The Reduced Spectral Fluorescence Spectroscopy (RSFS) device according to the present invention takes advantage of the fact, that ex/em pairs have been selected so that only the most significant parts of the SFS plots are measured to describe a parameter of a medium.

The device according to the present invention does not have any moving parts which makes the system more robust. No adjustments of the measurement system are needed prior the measurement, mobile and effective way of making RSFS is possible.

To overcome the known quenching problem which single wavelength fluorimeters have, the device according to the present invention measures multiple but only the most significant EX/EM pairs. Correlation databases for describing a medium, for example milk fat content; capsaicin content in chili peppers, are built based on RSFS measurements with the help of multivariate data analytical tools.

SFS measurements involve special measurement conditions, usually complete separation of ambient light is necessary. The present invention uses modulated light which makes fluorescence spectroscopy possible at the presence of ambient light. No special measurement conditions such as cuvettes, measurement chambers, are needed.

RSFS reduces the number of measurement points to 4, no scanning as laboratory fluorescence measurement involves, is not needed. This makes the fluorescence spectroscopy measurement by the present device and method possible in real-time.

The present device uses 4 diodes as an excitation source. The excitation light from diodes is filtered by optical bandpass filters which enables to narrow the excitation spectrum. The light after bandpass filters is monochromatic for each Ex wavelength. Four excitative monochromatic lights are carried to the substance by probe made from optical fibers. Four monochromatic lights excite dedicated fluorophores in the medium and emission spectrum is generated by the medium.

The emitted light is collected and transferred to the surface of photomultiplier with the set of optical fibers. The wavelength selective properties of photomultiplier are achieved by set of bandpass filters installed in front of photomultiplier and modulation characteristics of the excitative light. Four excitative diodes are modulated with different modulation characteristics. The emission spectra has the same modulation characteristics. So the photomultiplier can separate the emission spectra of the medium based on excitation light modulation properties (the emission light has the same modulation as the excitation as one is caused by another). Prechosen EX/EM filter pairs together with modulation enables to form a signal readout matrix (Table 1).

**Table 1 Signal Readout matrix**

| | Modulation filter 1 (Photomultiplier looks for signal with a modulation generated by diode 1 | Modulation filter 2 (Photomultiplier looks for signal with a modulation generated by diode 2 | Modulation filter 3 (Photomultiplier looks for signal with a modulation generated by diode 3 | Modulation filter 4 (Photomultiplier looks for signal with a modulation generated by diode 4 |
|---|---|---|---|---|
| EX/EM filter pair1 | Photomultiplier signal readout for EX/EM 1 | No signal | No signal | No signal |
| EX/EM filter pair2 | No signal | Photomultiplier signal readout for EX/EM2 | No signal | No signal |
| EX/EM filter pair3 | No signal | No signal | Photomultiplier signal readout for EX/EM3 | No signal |
| EX/EM filter pair4 | No signal | No signal | No signal | Photomultiplier signal readout for EX/EM4 |

The wavelengths of 4 LED diodes combined with band bass filters on excitation side and segmented bandpass filter with 4 sectors in front of photomultiplier on the emission side are chosen as most significant EX/EM pairs which characterize the correlation to be measured. These EX/EM pairs are chosen with the help of laboratory devices which measure large no. of EM/EX points and multivariate statistical tools suggest the most significant EX/EM pairs. In most cases where SFS spectroscopy is applicable 4 most significant EX/EM pairs has most the correlation information.

### Brief description of the drawings

The present invention is explained more precisely with references to figures added, where
Figure 1 describes a RSFS measurement device according to the present invention;
Figure 2.1. describes a cross-section A-A of glass fibers from figure 1 of the combined EX/EM probe configuration for centralized distribution of excitation light;
Figure 2.2. describes a cross-section A-A of glass fibers from figure 1 of the combined EX/EM probe for even distribution of excitation light;
Figure 3 describes a cross-section B-B from figure1, an emission band pass filter system.

### Detailed description of the invention

The device according to the present invention for Reduced Spectral Fluorescence Spectroscopy (RSFS) measurement comprises housing 1, multiple four excitation light emission diodes 2 combined with multiple of bandpass excitation filters 3 to produce monochromatic excitative wavelengths which are carried to the sample by excitation optical fibers 4, optical probe-to-sample distance detector 10 enabling to offset the error coming from measurement distance variation, optical bandpass emission filters 6 combined with glass emission fibers 5 carrying emission light, to the photomultiplier 7, EX/EM probe 8, which is formed of excitation optical fibers 4 together with emission fibers 5, and computing and communication device 9 (for example programmable digital signal processor).

In the preferred embodiment of the present invention, four excitation light emission diodes 2 and four bandpass excitation filters 3 are used.

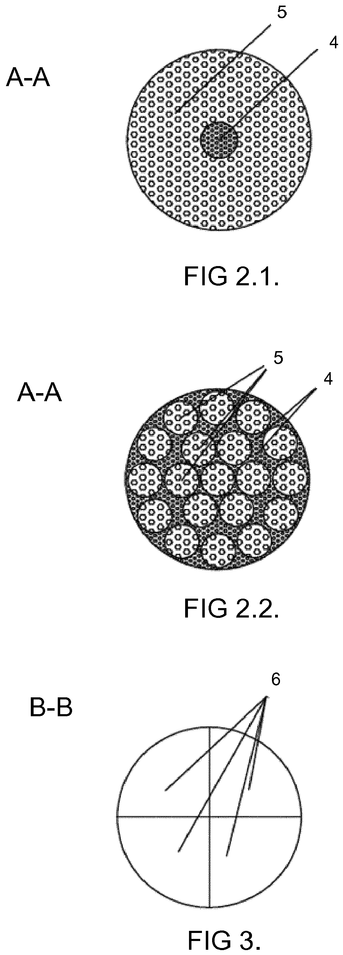

Fiber placement are described in figures 2-1 and 2-2. Emission filter 6 configuration is shown in figure 3. All connections to different parts of the device are static, no moving parts are used in construction.

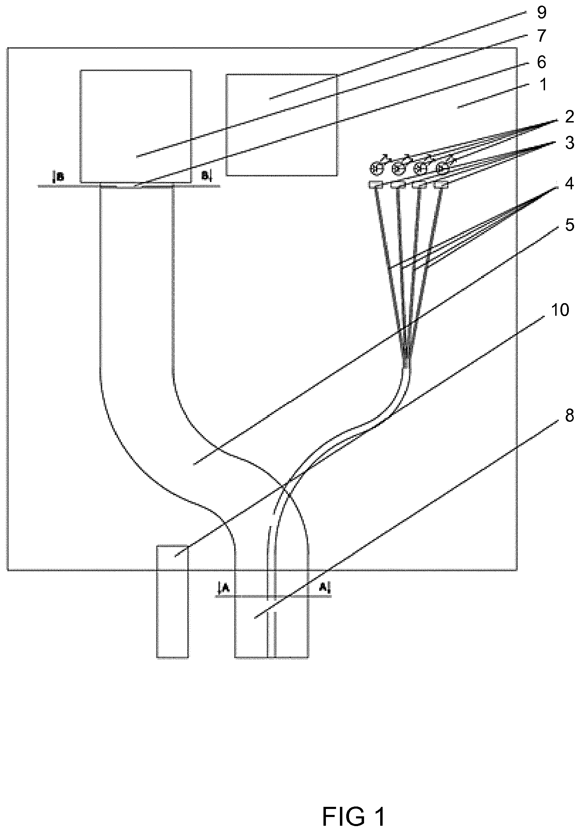

In the preferred embodiment, shown on fig 1, the device according to the present invention comprises four excitation filters 3 F1-F4, four excitation diodes 2 D1-D4, and four emission filters 6 F5-F8. Four excitation filters 3 F1-F4, four excitation diodes 2 D1-D4, and four emission filters 6 F5-F8 are exchangeable, wherein changeable frequency parameters are achieved by a changeable diode and filter sets. Sample is excited with light coming from excitation diodes with four different EX wavelengths, the emitting light from sample is gathered with optical fibers 5 which lead to photomultiplier 7. In front of the photomultiplier there are Emission bandpass optical F5-F8 filters 6. Programmable digital signal processor 9 comprising modulation filter in signal chain and communication ports is controlling the excitation diodes modulation and collects optically filtered and modelled signal readout from photomultiplier to measure fluorescence with the presence of backlight. Combined EX/EM probe 8 comprises both excitation fibers 4 and emission fibers 5. Also, the probe-to-sample distance sensor 10 is added so the distance can be considered during measurement.

Simultaneous and continuous measurement, which enables continuous operation control, is achieved by configuration of EX/EM probe 8 and filter system comprising excitation filters 3 and emission filters 6.

The device according to the present invention performs Reduced Spectral Fluorescence Spectroscopy. EX/EM pairs, detecting the emission light by photomultiplier; Excitation and emission pairs are formed in conjunction with filters and modulation of the excitation light. Table 1 describes the measurement matrix which is formed which enables the photomultiplier to distinguish between different EX/EM pairs.

The method according to the present invention for reduced fluorescence spectroscopy using a device comprising housing 1, multiple of excitation diodes 2 combined with multiple of bandpass excitation filters 3, optical fibers carrying excitation light 4, glass fibers carrying emission light 5, (together excitation and emission fibers form combined EX/EM probe 8 with a different fiber configuration), emission filters 6, photomultiplier 7, computing and communication device 9, optical sample distance detector 10, comprises following steps:
- preselecting EX diodes frequencies 2, EX filters 3 and EM filters 6 frequencies;
- selecting most significant EX/EM pairs;
- specifying the wavelengths of EX diodes 2 together with bandpass excitation filters 3 values to have monochromatic excitative light;
- setting the wavelengths of bandpass emission EM filters 6;
- providing excitation energy by excitation diodes 2;
- modulating the signal by using amplitude modulation;
- guiding the light to the surface of excitation bandpass filters;
- setting four monochromatic waves for excitation of the medium;
- guiding monochromatic waves to the sample surface with glass or plastic fibers 4;
- exciting the sample with monochromatic EX wavelengths;
- collecting excitation induced emission light together with ambient light from the surface of the sample by emission fibers 5;
- guiding the light by emission fibers 5 on the surface of emission filters 6;
- filtering the ambient light out;
- forming excitation and emission pairs in conjunction with filters and modulation of the excitation light;
- detecting the emission light by photomultiplier;
- based on measured RSFS calculate parameter of a medium.
   a) The step of preselecting EX diodes frequencies (2) and EX filters 3 and EM filters 6 bandpass frequencies and four most significant EX/EM pairs which describe the parameter of the medium most are selected based on laboratory research, specifies the wavelengths of EX diodes 2 together with bandpass excitation filters 3 values to have a monochromatic excitation light. Bandpass emission filters 6 wavelengths are also set at this stage so that EX/EM pairs are formed. These characteristic EX/EM frequencies describe medium parameters the most, used EX/EM frequencies are specified from applied PCA, LSA or PARAFAC analyses to the SFS spectrums of a medium with a laboratory device where complete spectral fluorescence spectrogram is measured. These prechosen EX/EM frequencies are used to measure RSFS with a device according to the present invention.
   b) By providing excitation energy by excitation diodes 2, diode will provide modulated light with a certain EX wavelength and amplitude modulation is used to modulate the signal. This light goes to the surface of excitation bandpass filters.
   c) By narrowing the wavelength window of diode spectrum with an optical bandpass filter 3, the diode spectrum is usually 10-15 nm wide bandpass filter, which narrows the excitation spectrum from 15 nm to 2 nm. Four monochromatic waves for excitation of the medium are set after light passing the filters.
   d) By guiding monochromatic waves to the sample surface with optical fibers 4, the sample is excited with monochromatic EX wavelengths.
   e) In step of gathering the emission light by another set of fibers 5, the excitation induced emitted light from the sample is gathered by emission fibers which guide the light on the surface of emission filters.
   f) In step of choosing the properties of emission filter, the light is collected from the surface of the sample comprises of the response to the modulated excitation monochromatic light and ambient light. This emission light has wide spectra, which comprises all the emitted light. Emission bandpass filter parameters are chosen so that these let throw only light with EM wavelengths. At this moment four modulated monochromatic waves reach at the surface of photomultiplier. Ambient light is filtered out mathematically as it is constant and unmodulated.
   g) By forming EX/EM pairs, the emission light by photomultiplier is detected and excitation and emission pairs are formed in conjunction with filters and modulation of the excitation light. Measurement matrix described in Table 1 is formed.
   h) By measuring RSFS spectrogram at this point we can calculate various properties of the medium based on correlation databases are calculated. For example, calculate milk fat content or capsaicin content in chili peppers.

The set of LED diodes provide excitation energy. Each diode has a dedicated wavelength, chosen to match the excitation energy needed for the fluorophore (for example tryptophan ex/em pair in milk related food matrix is λ_{max_emission}=280nm/ λₘₐₓ__{excitation}=357nm). The other option is that excitation and emission wavelengths are chosen from correlation databases to measure a certain property of material. At this case 4 most significant ex/em wavelength pairs contributing to the strength of correlation between SFS measurement and parameter of the medium are chosen. Multi-dimensional data analysis tool, for example Principal Component Analysis (PCA), PARAllel FACtor(PARAFAC) or similar, is used to verify the EX/EM pairs that describe a correlation of a medium property. For example, measurement of capsaicin content in chili pepper 4 most significant EX/EM pairs based on PCA are 270nm/295nm 280nm/325nm 310nm/415nm 345nm/465nm but these don't match to any certain fluorophore EX/EM pair. These kinds of most significant EX/EM pairs can be set if the parameter of the medium is measurable with spectral fluorescence spectroscopy.

Excitation LEDs together with excitation filters and emission filters are changeable according to the material measured. For example, if to measure capsaicin content of chili pepper the EX/EM pairs would be 270nm/295nm 280nm/325nm 310nm/415nm 345nm/465nm or for milk fat EX/EM 250/325 330/410 340/480 345/465. Furthermore, all-purpose EX/EM pairs can be calculated. This kind of all-purpose solution can be used in mobile phone additional device to perform general purpose measurements with lower correlation coefficients.

The distance detector 10 allows to offset the error coming from measuring distance difference.

The excitation light is guided to the sample through excitation optical fibers 4 (for example glass or plastic fibers). The arrangement of the optical fibers (Figure 2) is dependent of the goal of measurement and the probe configuration according to the area of the sample is adjusted. In case of homogenous materials (for example milk) centralized probe shown in Figure 2.1. A-A 1 is used for better S/N ratio. Not homogeneous materials (for example herbs) wide excitation of optical fibers shown in Figure 2.2 A-A 2 is used for even distribution of excitative light.

The emission light is gathered by another set of fibers 4. In centralized configuration, most of dissipated light is gathered. This is because the excitation area is small, small amount of sample is excited, the emission probes collect most of the dissipated light originated from the sample.

In even distribution of fibers larger amount of sample is illuminated with excitation energy which enables to average the signal, this is used for not homogenized samples of a medium. Emission light gathering glass fibers are placed evenly on the surface of probe.

## Claims

1. A device for simultaneous and continuous measurements of reduced fluorescence spectroscopy combining the probe and filter system, is **characterized by** that, the device comprises housing (1), multiple of excitation diodes (2) combined with multiple of bandpass excitation filters (3) to produce the excitative energy which is carried to the sample by excitation optical fibers (4), optical probe-to-sample distance detector (10) enabling to offset the error coming from measurement distance variation, optical bandpass emission filters (6) combined with glass emission fibers (5) carrying emission light to the photomultiplier (7), EX/EM probe (8), which is formed of excitation fibers (4) together with emission fibers 5, programmable digital signal processor (9).

2. The device according to the claim 1, **characterized by** that, the connections of different parts of the device are static without moving parts in construction.

3. The device according to the claim 1, **characterized by** that, the excitation filters (3), excitation diodes (2) and emission filters (6) are changeable, wherein the changeable frequency parameters are achieved by a changeable diode and filter sets.

4. The device according to the claim 1, **characterized by** that, the programmable digital signal processor (9) comprising modulation filter in signal chain and communication ports is controlling the excitation diodes modulation and collects optically filtered and modelled signal readout from photomultiplier to measure fluorescence with the presence of backlight.

5. The device according to the claim 1, **characterized by** that, the EX/EM probe (8) comprises excitation fibers (4) and emission fibers (5).

6. The device according to the claim 1, **characterized by** that, the excitation and emission pairs are formed in conjunction with filters and modulation of the excitation light.

7. The device according to the claim 1, **characterized by** that, the EX/EM probe (8) is combined with filter system comprising excitation filters (3) and emission filters (6).

8. The device according to the claim 1, **characterized by** that, the probe configuration is centralized to measure narrow area of sample.

9. The device according to the claim 1, **characterized by** that, the probe configuration is even to measure wide area of sample.

10. A method according to the present invention for reduced fluorescence spectroscopy using a device comprising housing (1), multiple of excitation diodes (2) combined with multiple of bandpass excitation filters (3), optical fibers carrying excitation light 4, glass fibers carrying emission light (5), together excitation and emission fibers form combined EX/EM probe (8) with a different fiber configuration (8), emission filters (6), photomultiplier (7), computing and communication device (9), optical sample distance detector (10), comprises following steps:
- preselecting EX diodes frequencies (2), EX filters (3) and EM filters (6) frequencies;
- selecting most significant EX/EM pairs;
- specifying the wavelengths of EX diodes (2) together with bandpass excitation filters (3) values to have monochromatic excitative light;
- setting the wavelengths of bandpass emission EM filters (6);
- providing excitation energy by excitation diodes (2);
- modulating the signal by using amplitude modulation;
- guiding the light to the surface of excitation bandpass filters;
- setting four monochromatic waves for excitation of the medium;
- guiding monochromatic waves to the sample surface with glass or plastic fibers (4);
- exciting the sample with monochromatic EX wavelengths;
- collecting excitation induced emission light together with ambient light from the surface of the sample by emission fibers (5);
- guiding the light by emission fibers (5) on the surface of emission filters (6);
- filtering the ambient light out;
- forming excitation and emission pairs in conjunction with filters and modulation of the excitation light;
- detecting the emission light by photomultiplier;
- based on measured RSFS calculate parameter of a medium.

11. The method according to the claim 10, **characterized by** that, the probe configuration is centralized to measure narrow area of sample.

12. The method according to the claim 10, **characterized by** that, the probe configuration is even to measure wide area of sample.

13. The method according to the claim 10, **characterized by** that, each spectrum coming from excitation diodes (2) is filtered with an optical bandpass filter (3) which narrows the excitation light spectrum from 15 nm to 2nm.
